(19) Europäisches Patentamt
European Patent Office
Office européen des brevets

(11) Publication number: **0 511 563 A1**

# (12) EUROPEAN PATENT APPLICATION

(21) Application number: **92106655.1**

(22) Date of filing: **16.04.92**

(51) Int. Cl.$^5$: **C07H 15/20**, C07H 17/04, C07F 9/12

(30) Priority: **29.04.91 US 693981**
**20.02.92 US 834954**

(43) Date of publication of application:
**04.11.92 Bulletin 92/45**

(84) Designated Contracting States:
**AT BE CH DE DK ES FR GB GR IT LI LU MC NL PT SE**

(71) Applicant: **Bristol-Myers Squibb Company**
**345 Park Avenue**
**New York, N.Y. 10154(US)**

(72) Inventor: **Saulnier, Mark G.**
**57 Baldwin Court**
**Newington, CT 0611(US)**
Inventor: **Kadow, John F.**
**9 Ouarry Run Court**
**Wallingford, CT 06492(US)**
Inventor: **Langley, David R.**
**56 Gravel Street**
**Meriden, CT 06450(US)**
Inventor: **Tun, Min Min**
**137 Cottage Street E5**
**New Haven, CT 06511(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Process for the preparation of 4'-demethylepipodophyllotoxin glucoside 4'-phosphates.

(57) The present invention relates to a process for preparing 4'-demethylepipodophyllotoxin glucoside 4'-phosphate, which comprise reacting a protected 4'-demethylpodophyllotoxin with a protected glucopyranose in the presence of a Lewis acid, followed by deprotection.

EP 0 511 563 A1

## BACKGROUND OF THE INVENTION

### 1. Field of the Invention

The present invention relates to a novel process for the preparation of antitumor compounds, as well as to the novel intermediates produced in said process. More particularly, the novel process and intermediates are directed to the preparation of 4'-demethylepipodophyllotoxin glucoside 4'-phosphates.

### 2. Background Art

Etoposide and teniposide, two 4'-demethylepipodophyllotoxin glucoside derivatives, are presently widely used in clinical therapy of cancer and etoposide is currently approved in the United States for the treatment of small cell lung cancer and testicular cancer. However, these compounds have very low water solubility rendering them difficult to formulate into suitable pharmaceutical dosage forms.

US Patent 4,904,768 discloses water soluble prodrugs of 4'-demethylepipodophyllotoxin glucoside derivatives bearing a 4'-phosphate group, as exemplified by etoposide 4'-phosphate. Etoposide phosphate was prepared by reacting etoposide with phosphorous oxychloride followed by hydrolysis, or by reacting etoposide with diphenyl chlorophosphate followed by hydrogenation to remove the phenyl groups.

Etoposide 4'-phosphate is also reported in Japanese Kokai 63/192,793 (published August 10, 1988) and it was prepared by reacting 2'',3''-bis-O-(2,2,2-trichloroethoxycarbonyl)etoposide with phosphorous oxychloride followed by hydrolysis, and then removal of the sugar hydroxy protecting groups by treatment with zinc.

The starting materials used in the above processes are obtained by condensing 4'-protected 4'-demethylepipodophyllotoxin with hydroxy-protected ethylidene-$\beta$-D-glucopyranose, and subsequently removing at least the 4'-protecting group. Surprisingly and unexpectedly, the present inventors found that the 4'-phenolic hydroxy group of 4'-demethylepipodophyllotoxin may be protected in the form of a phosphate triester which, following condensation with the glucopyranosyl moiety, may be cleaved to provide etoposide 4'-phosphate directly. The present process eliminates the necessity of separately protecting and deprotecting the 4'-phenolic hydroxy group of 4'-demethylepipodophyllotoxin, and therefore represents a more economical and efficient route over the other known art methods for the preparation of etoposide 4'-phosphate.

## SUMMARY OF THE INVENTION

The present invention relates to a process for preparing a compound of formula (IV)

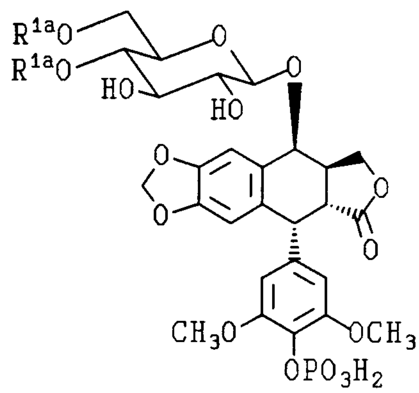

(IV)

wherein the two $R^{1a}$ groups together represent $C_{1-5}$ alkylidene, or a pharmaceutically acceptable salt thereof, or a solvate thereof, which comprises: reacting a compound of formula (II)

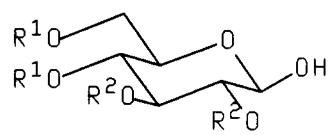

(II)

wherein $R^1$ is hydroxy protecting group, or the two $R^1$ groups together represent $C_{1-5}$ alkylidene, and $R^2$ is hydroxy protecting group, with a compound of formula (III)

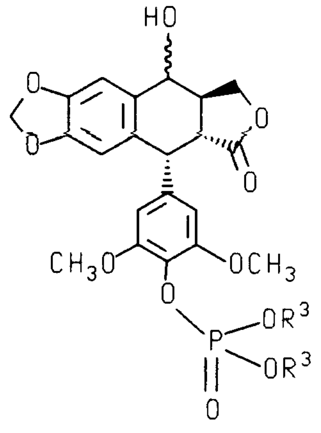

( I I I )

wherein $R^3$ is phosphate protecting group, in the presence of a Lewis acid to form a compound of formula (I);

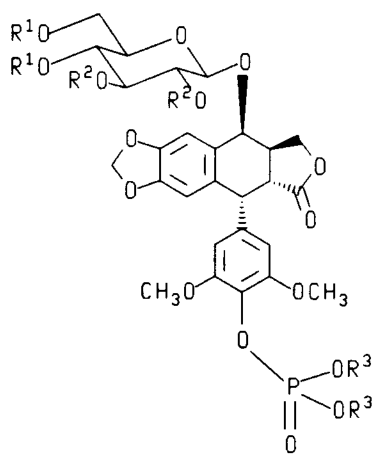

( I )

removing the hydroxy protecting groups, and where $R^1$ is hydroxy protecting group, reacting the resultant product with a carbonyl compound having one to five carbon atoms or an acetal or a ketal equivalent thereof; and removing the phosphate protecting groups.

Another aspect of the present invention provides a novel process for the preparation of a protected 4'-demethylepipodophyllotoxin glucoside 4'-phosphate intermediate of formula (I) which comprises reacting a hydroxy protected glucopyranose of formula (II) and a phosphate protected 4'-demethylpodophyllotoxin 4'-phosphate, in the presence of a Lewis acid.

The present invention also contemplates the novel compounds of formulas (I) and (III).

## DETAILED DESCRIPTION OF THE INVENTION

The present invention provides an improved method for preparing 4'-demethylepipodophyllotoxin glucoside 4'-phosphates, pharmaceutically acceptable salts thereof, or solvates thereof; in particular, the process is suitable for the preparation of etoposide 4'-phosphate including its pharmaceutically acceptable salts and solvates. As used herein, "pharmaceutically acceptable salts" include mono- or di-alkali metal salts, and alkaline earth metal salts; preferably the pharmaceutically acceptable salt is the disodium salt. "Solvates" are formed by crystallization or recrystallization from organic solvents such as ethanol or from water (hydrates). The term "alkylidene" includes straight and branched carbon chains, for example ethylidene, propylidene and isopropylidene. The term "4'-demethylpodophyllotoxin," unless otherwise specified, will be understood to encompass 4'-demethylpodophyllotoxin and 4'-demethylepipodophyllotoxin, individually and as a mixture thereof.

The process of the present invention comprises reacting a phosphate-protected 4'-demethylpodophyllotoxin of formula (III) with a hydroxy-protected glucopyranose of formula (II) in the presence of a Lewis acid to provide an intermediate of formula (I). The hydroxy and phosphate protecting groups on the intermediate are then removed to give 4'-demethylepipodophyllotoxin glucoside 4'-phosphate of formula (IV) directly, where the two $R^1$ groups together represent $C_{1-5}$ alkylidene; or where $R^1$ is hydroxy protecting group, the product is reacted with an appropriate carbonyl compound or an acetal or ketal equivalent thereof to give compound of formula (IV).

In compounds of formulas (I) and (III), the phosphate protecting group may be any that is known in the art, examples of which include, but are not limited to, 2,2,2-trichloroethyl, phenyl, substituted phenyl, benzyl and substituted benzyl; the substituent may be one or more groups selected from methoxy, nitro, and methyl. Preferably, the phosphate protecting group is phenyl or benzyl; and most preferably it is benzyl.

In compounds of formulas (I) and (II) the hydroxy protecting group may be any that is conventionally used and include, but is not limited to, ethers such as methyl, t-butyl, benzyl, p-methoxybenzyl, p-nitrobenzyl, allyl, trityl, methoxymethyl, methoxyethoxymethyl, ethoxyethyl, tetrahydropyranyl, tetrahydrothiopyranyl, and trialkylsilyl ethers such as trimethylsilyl ether and t-butyldimethylsilyl ether; esters such as benzoyl, acetyl, phenylacetyl, formyl, mono-, di-, and

trihaloacetyl such as chloroacetyl, dichloroacetyl, trichloroacetyl, trifluoroacetyl; and carbonates such as methyl, ethyl, 2,2,2-trichloroethyl, benzyl, and p-nitrophenyl. More preferably the hydroxy protecting group is one commonly used in podophyllotoxin chemistry; examples that may be mentioned include, but are not limited to, benzyl; esters such as $C_{1-3}$alkanoyl and halogenated $C_{2-3}$alkanoyl groups, e.g. formyl, acetyl, mono-, di-, and trihaloacetyl; and $C_{1-3}$alkyl or halogenated $C_{1-3}$alkyl carbonates such as ethyl, and mono-, di-, and trihaloethyl carbonates. Halogen includes fluorine, bromine, chlorine and iodine. Most preferably the hydroxy protecting group is 2,2,2-trichloroethyl carbonate (i.e. the 2,2,2-trichloro-ethoxycarbonyl group).

Compounds of formula (III) may be obtained by reacting 4'-demethylpodophyllotoxin with a chlorophosphate $ClP(O)(OR^3)_2$ wherein $R^3$ is as previously defined. The reaction is carried out in an inert organic solvent such as acetonitrile at ambient temperature and in the presence of an acid scavenger, e.g. a tertiary amine base such as diisopropyl ethylamine. Illustrative of this general process is the reaction of 4'-demethylepipodophyllotoxin with diphenyl chlorophosphate in the presence of diisopropylethylamine in acetonitrile at room temperature to provide 4'-demethylepipodophyllotoxin 4'-diphenylphosphate.

The stereochemical configuration at position 1 of the starting material of formula (III) is not critical as the condensation of a compound of formula (II) with a compound of formula (III) under the present conditions generally results in a product having the desired configuration at position 1, i.e. an epipodophyllotoxin. Thus the starting material may be phosphate-protected 4'-demethylepipodophyllotoxin, phosphate-protected 4'-demethylpodophyllotoxin, or a mixture thereof.

Tetra-O-protected β-D-glucopyranose derivatives of formula (II) and methods for their preparation are generally known in the art. Compounds of formula (II) wherein the two $R^1$ groups together represent $C_{1-5}$alkylidene may also be prepared by known methods, for example, by reacting glucose with an appropriate carbonyl compound, or an acetal or ketal equivalent thereof, in the presence of an acid catalyst to form the cyclic acetal, and subsequently introducing the $R^2$ hydroxy protecting groups. The carbonyl compound may be for example acetaldehyde, propionaldehyde, or acetone, and equivalents thereof such as paraldehyde, acetaldehyde dimethyl acetal, and acetone dimethyl acetal. Thus, as an example, D-glucose is treated with paraldehyde in the presence of a mineral acid, e.g. sulfuric acid to provide 4,6-O-ethylidene-D-glucose which is treated with sodium hydroxide and then benzylchloroformate to convert the anomeric hydroxy group to the benzyl carbonate. The compound formed is treated with 2,2,2-trichloroethyl chloroformate, and the resulting product is subject to catalytic hydrogenation to provide 4,6-O-ethylidene-2,3-bis-O-(2,2,2-trichloroethoxycarbonyl)-β-D-glucopyranose. This compound is also disclosed in Japanese Kokai J58-225,096.

The coupling of the protected glucopyranose of formula (II) and the protected 4'-demethylpodophyllotoxin 4'-phosphate of formula (III) to form a compound of formula (I) is carried out in an inert organic solvent and in the presence of a Lewis acid, preferably boron trifluoride etherate. The solvent is preferably a halogenated hydrocarbon such as methylene chloride, dichloroethane or chloroform. The reaction temperature is typically below 0 °C and suitably about -15 to about -25 °C.

The hydroxy and phosphate protecting groups of compounds of formula (I) are then removed to provide the corresponding 4'-demethylepipodophyllotoxin glucoside 4'-phosphate derivatives. Deprotection may be effected by methods generally known in the art, and examples that may be mentioned include hydrolysis, hydrogenation, hydrogenolysis, reduction, and the like. For example, the 2,2,2-trichloroethoxycarbonyl hydroxy protecting group may be removed with a zinc reagent such as zinc and acetic acid; the phenyl and benzyl phosphate protecting groups may be removed by catalytic hydrogenolysis, and the benzyl group may also be removed by catalytic transfer hydrogenation using palladium on carbon and a reagent such as 1,4-cyclohexadiene and 1-methyl-1,4-cyclohexadiene. Depending on the nature of the protecting groups the deprotection may be effected sequentially in which case the order of removal is not particularly critical, or if suitable, the protecting groups are removed in one step.

Where $R^1$ is hydroxy protecting group, the compound produced after removing the hydroxy protecting group is reacted with an appropriate carbonyl compound, e.g. acetaldehyde, propionaldehyde, acetone, or an acetal or ketal equivalent thereof such as paraldehyde, acetaldehyde dimethyl acetal, and acetone dimethyl acetal, in the presence of an acid catalyst to form the corresponding compound of formula (IV).

In one specific embodiment illustrative of the present process, 4'-demethylepipodophyllotoxin 4'-diphenylphosphate is reacted with 4,6-O-ethylidene-2,3-bis-O-(2,2,2-trichloroethoxycarbonyl)-D-glucopyranose in dichloroethane at -20 °C in the presence of boron trifluoride etherate to give 2,3-bis-O-(2,2,2-trichloroethoxycarbonyl)etoposide 4'-diphenylphosphate. Treatment with zinc and acetic acid to remove the 2,2,2-trichloroethoxycarbonyl groups,

followed by catalytic hydrogenolysis to remove the phenyl groups, provides etoposide 4'-phosphate. In another example, the benzyl group is used as phosphate protecting group, and is removed by catalytic transfer hydrogenation with e.g. palladium on carbon and 1-methyl-1,4-cyclohexadiene.

The 4'-demethylepipodophyllotoxin glucoside 4'-phosphate of formula (IV) may be converted to its pharmaceutically acceptable salt by contacting it with a source of the appropriate cation; for example treating it with a base such as sodium carbonate results in the formation of the sodium salt thereof. Solvates of the 4'-demethylepipodophyllotoxin glucoside 4'-phosphate of formula (IV) may also be obtained by crystallizing or recrystallizing from organic solvents or water; thus, for example, the diethanolate of etoposide 4'-phosphate may be obtained from a saturated solution of etoposide 4'-phosphate in ethanol containing solvent system.

Another aspect of the present invention provides the novel compounds of formula (I)

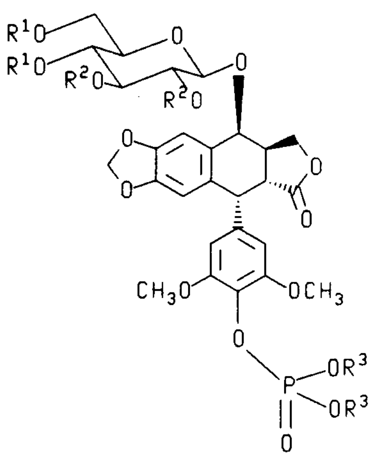

(I)

wherein $R^1$ is a hydroxy protecting group, or the two $R^1$ groups together represent $C_{1-5}$alkylidene; $R^2$ is a hydroxy protecting group; and $R^3$ is a phosphate protecting group. Preferably $R^1$ is $C_{1-5}$alkylidene, $R^2$ is a hydroxy protecting group selected from benzyl, $C_{1-3}$alkanoyl, halogenated $C_{1-3}$alkanoyl, $C_{1-3}$alkoxycarbonyl and halogenated $C_{1-3}$alkoxycarbonyl, and $R^3$ is selected from phenyl, substituted phenyl, benzyl and substituted benzyl. More preferably, $R^2$ is halogenated ethoxycarbonyl, and $R^3$ is phenyl or benzyl; most preferably, the two $R^1$ together represent ethylidene, $R^2$ is 2,2,2-trichloroethoxycarbonyl, and $R^3$ is benzyl.

Another aspect of the present invention provides novel compounds of the formula (III)

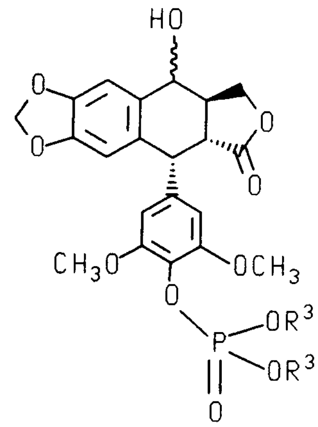

(III)

wherein $R^3$ is a phosphate protecting group; preferably $R^3$ is selected from phenyl, substituted phenyl, benzyl, substituted benzyl, and 2,2,2-trichloroethyl; more preferably $R^3$ is phenyl or benzyl; most preferably $R^3$ is benzyl.

The following examples are offered in order to more fully illustrate the present invention and shall not be construed to limit the scope of the invention in any manner.

## Example 1

### 4'-Demethylepipodophyllotoxin-4'-diphenylphosphate (Compound 1)

To a solution of 4'-demethylepipodophyllotoxin (1.44g), prepared from podophyllotoxin as described (Helv. Chim. Acta. 152, p. 944, 1969), in dry acetonitrile (25 ml) was added diisopropylethylamine (941 $\mu$L) and diphenyl chlorophosphate (821 $\mu$L). The mixture was stirred at room temperature for 16 h and concentrated in vacuo. The residue was dissolved in $CH_2Cl_2$ and washed with $H_2O$ and brine. Purification was effected by medium pressure chromatography on silica gel using 2% MeOH in $CH_2Cl_2$ followed by crystallization from EtOAc in hexane. The unoptimized yield of pure 1 was 700 mg (ca 30%).

$^1$H-NMR ($CDCl_3$) $\delta$ 7.35-7.15 (m,10H), 6.81(s,1H), 6.43(s,1H), 6.23 (s,2H), 5.94 (d,2H), 4.78 (t,1H), 4.54 (d,1H, J = 5Hz), 4.35-4.20 (m,2H), 3.48 (s,6H), 3.24 (dd,1H,J = 13.2 and 5Hz), 2.73-2.63 (m, 1H), 1.97 (d,1H,OH).

HRMS calcd. for $C_{33}H_{30}O_{11}P$ (M + H):
633.1526
Found:
633.1532

Example 2

4'-Demethylepipodophyllotoxin-4'-dibenzylphosphate (Compound 2)

Dibenzyl chlorophosphate (6 equiv) was freshly prepared from equimolar amounts of N-chlorosuccinimide and dibenzyl phosphite refluxing in $CH_2Cl_2$ (150 ml) for 20 min. This mixture was filtered and the filtrate was added to a solution of 4'-demethylepipodophyllotoxin (1.22 g), diisopropylethylamine (1.7ml), and 4-dimethylaminopyridine (37mg) in dry acetonitrile. The mixture was stirred for 16 h and then partitioned with $CH_2Cl_2$ and saturated $NaHCO_3$. The organic extracts were washed with $H_2O$ and brine and dried ($Na_2SO_4$).Purification was effected by flash chromatography on silica gel using 1% MeOH in $CH_2Cl_2$ followed by crystallization from EtOAc, 10% MeOH in $CH_2Cl_2$ followed by crystallization from EtOAc, 10% MeOH in $CH_2Cl_2$, and hexane. The unoptimized yield of pure 2 was 760 mg (ca 38%).

$^1$H-NMR ($CDCl_3$) $\delta$ 7.37-7.27 (m,10H), 6.83 (s,1H), 6.49 (s,1H), 6.27 (s,2H), 5.96 (d,2H), 5.25-5.17 (m,4H), 4.82 (d,1H,J=3.3Hz), 4.60 (d,1H,J=5Hz), 4.40-4.30 (m,2H), 3.62 (s,6H), 3.28 (dd,1H,J=13 and 5Hz), 2.77-2.68 (m,1H).

Mass Spectrum, m/e=661 ($M^+$ +H).

Example 3

2'',3''-bis-O-[2,2,2-trichloroethoxy)carbonyl]-etoposide -4'-diphenylphosphate (Compound 3)

To a suspension of Compound 1 (50mg) and 4,6-O-ethylidene-2,3-bis-O-[(2,2,2-trichloroethoxy)-carbonyl]-$\beta$-D-glucopyranose (45mg) in dry 1,2-dichloroethane (0.5ml) at -20°C under argon was added boron trifluoride etherate (27 $\mu$l) dropwise over 5 min. After 2 h at -20°C, the mixture was quenched with pyridine (50 $\mu$l) and allowed to warm to room temperature. The mixture was diluted with $CH_2Cl_2$ (20 ml) and washed with cold 1 N HCl (2x), $H_2O$, and brine and dried ($MgSO_4$). Purification by preparative TLC using 1% MeOH in $CH_2Cl_2$ gave 74.5 mg (80.5%) of pure title compound, 3.

IR (KBr) 1778, 1600, 1488, 1284, 1260, 1232, 1190, 1162, 1132, 958, 822, 774 $cm^{-1}$.

$^1$H-NMR ($CDCl_3$) $\delta$ 7.35-7.15 (m,10H), 6.72 (s,1H), 6.47 (s,1H), 6.15 (s,2H), 5.98 (s,2H), 4.88 (d,1H), 4.82-4.45 (m,7H), 4.35 (m,1H), 4.25-4.18 (m,2H), 3.80-3.49 (m,4H), 3.48 (s,6H), 3.41-3.37 (m,1H), 3.13 (dd, 1H, J=14.1 and 5.2 Hz), 2.86-2.76 (m,1H), 1.31 (d,3H,J=5Hz).

Mass Spectrum, m/e=1171 ($M^+$ +H).

Example 4

2'',3''-bis-O-[2,2,2-trichloroethoxy)carbonyl]-etoposide-4'-dibenzylphosphate (Compound 4)

The procedure described in Example 3 was repeated using Compound 2 (145 mg) and 4,6-O-ethylidene-2,3-bis-O-[2,2,2-trichloroethoxy)-carbonyl]-$\beta$-D-glucopyranose (135 mg) in dry 1,2-dichloroethane (1.5 ml) with boron trifluoride etherate (74 $\mu$L) to provide 240 mg of pure title compound, 4.

IR (KBr) 1768, 1599, 1505, 1485, 1458, 1420, 1381, 1338, 1260, 1229, 938, 818 $cm^{-1}$.

$^1$H-NMR ($CDCl_3$) 7.35-7.25 (m,10H), 6.75 (s,1H), 6.51 (s,1H), 6.22 (s,2H), 5.98 (s,2H), 5.24-5.14 (m,4H), 4.91 (d,1H), 4.83-4.47 (m,7H), 4.37 (m,1H), 4.24-4.20 (m,2H), 3.80-3.45 (m,4H), 3.62 (s,6H), 3.44-3.35 (m,1H), 3.15 (dd,1H,J=14 and 5.2 Hz), 2.88-2.78 (m,1H), 1.31 (d,3H,J=4.9Hz).

Mass Spectrum, m/e=1199 ($M^+$ +H).

Example 5

Etoposide 4'-diphenylphosphate.

To a solution of compound 3 (200 mg) in dry THF (8 ml) under $N_2$ at room temperature was added zinc dust (468 mg) followed by acetic acid (4.8 ml). The reaction mixture was sonicated at 25-30°C for 30 min and then stirred for 3 h at room temperature. The mixture was filtered through Celite and washed with $CH_2Cl_2$ (200 ml). The filtrate was washed with saturated $NaHCO_3$, $H_2O$ and brine, and dried ($MgSO_4$). Evaporation in vacuo followed by flash chromatography on silica gel using 0-3% MeOH in $CH_2Cl_2$ gave 116 mg (83%) of pure title compound.

The conversion of the title compound to etoposide 4'-phosphate is described in U.S. Patent 4,904,768.

Example 6

Etoposide 4'-dibenzylphosphate.

The procedure described in Example 5 was repeated using a solution of Compound 4 (152 mg) in dry dioxane (2ml), zinc dust (315 mg) and acetic acid (1.2 ml) to provide 126 mg (66%) of pure title compound.

Example 7

Etoposide 4'-phosphate.

A solution of 4'-dibenzylphosphate etoposide (500 g, 0.59 mole) in methanol (2 l) is added to a

suspension of 10% Pd/C (50 g) in methanol (1 l), and the suspension is heated to about 37°C. To this suspension is added slowly a solution of 1-methyl-1,4-cyclohexadiene (555 g, 660 ml, 5.89 mole, 10 eq) in methanol (1 l), and the suspension is stirred at 40 - 45°C until the reaction is complete as shown by TLC. The reaction mixture is filtered and the volume of the filtrate is adjusted to about 1 l by concentrating or adding additional methanol, the resulting solution is then added to absolute ethanol (4 l). The solution is seeded with etoposide 4'-phosphate reference standard and concentrated to about 2.5 l. Absolute ethanol (3.5 l) is added to the slurry and stirred at about 20°C for 18 - 72 hours. The solids are collected by filtration, washed with absolute ethanol (2 x 250 ml) and dried under vacuum for 18 hours at about 20°C to provide 300 - 350 g of the title compound as the diethanolate (80 - 90% yield).

Melting point: 141 - 150°C (lose solvent); 160 - 172°C (melt).

Ethanol residue: 11.8 % (by NMR); 13.2 % (by thermogravimetric analysis). Calc. for $C_{29}H_{33}O_{16}P.2C_2H_6O$ 12.1%.

Moisture content: 0.22% by Karl Fischer method

## Claims

1. A process for preparing a compound of formula (IV)

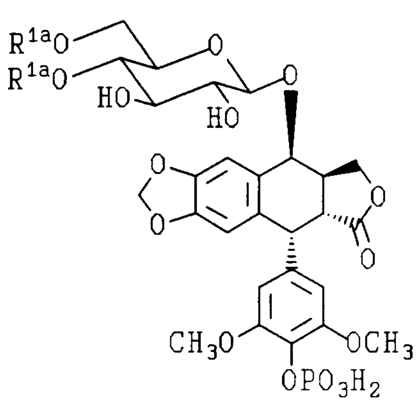

(IV)

wherein the two $R^{1a}$ groups together represent $C_{1-5}$ alkylidene, or a pharmaceutically acceptable salt thereof, or a solvate thereof, which comprises:

reacting a compound of formula (II)

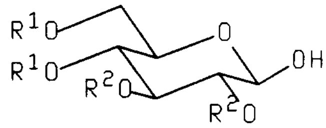

(II)

wherein $R^1$ is hydroxy protecting group, or the two $R^1$ groups together represent $C_{1-5}$ alkylidene, and $R^2$ is hydroxy protecting group, with a compound of formula (III)

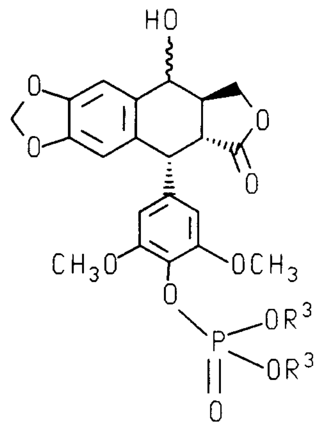

(III)

wherein $R^3$ is phosphate protecting group, in the presence of a Lewis acid to form a compound of formula (I);

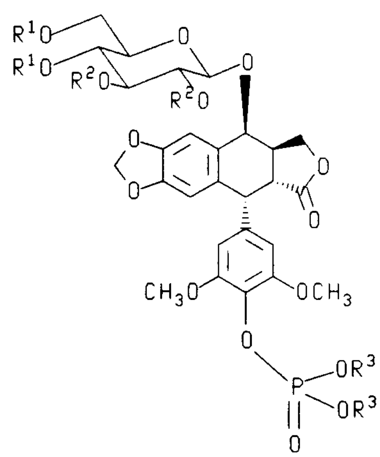

(I)

removing the hydroxy protecting groups,

and where $R^1$ is hydroxy protecting group, reacting the resultant product with a carbonyl compound having one to five carbon atoms or an acetal or a ketal equivalent thereof; removing the phosphate protecting groups.

**2.** A process according to Claim 1 for preparing a compound of formula (IV) wherein the two $R^1$ groups represent $C_{1-5}$alkylidene; $R^2$ is selected from the group consisting of benzyl, $C_{1-3}$alkanoyl, halogenated $C_{1-3}$alkanoyl, $C_{1-3}$alkoxycarbonyl and halogenated $C_{1-3}$alkoxycarbonyl; and $R^3$ is selected from the group consisting of phenyl, substituted phenyl, benzyl, substituted benzyl, and 2,2,2-trichloroethyl.

**3.** A process according to Claim 1 for preparing a compound of formula (IV) wherein the two $R^1$ groups represent ethylidene, $R^2$ is 2,2,2-trichloroethoxycarbonyl, and $R^3$ is phenyl or benzyl.

**4.** A process according to Claim 3 wherein $R^3$ is benzyl.

**5.** A process according to Claim 1 wherein said Lewis acid is boron trifluoride etherate.

**6.** A process according to Claim 2 wherein said Lewis acid is boron trifluoride etherate.

**7.** A process according to Claim 3 wherein said Lewis acid is boron trifluoride etherate.

**8.** A process according to Claim 4 wherein said Lewis acid is boron trifluoride etherate.

**9.** A process according to Claim 7 wherein said hydroxy protecting groups are removed with a zinc reagent, and said phosphate protecting groups are removed by catalytic hydrogenolysis.

**10.** A process according to Claim 8 wherein said hydroxy protecting groups are removed with a zinc reagent, and said phosphate protecting groups are removed by catalytic transfer hydrogenation.

**11.** A process for preparing a compound of formula (I)

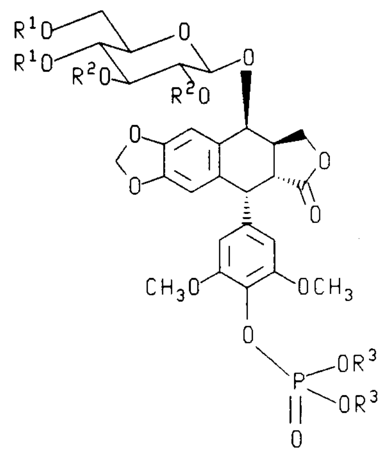

(I)

which comprises reacting a compound of formula (II)

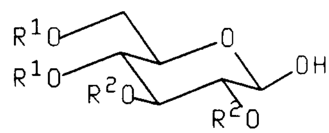

(II)

with a compound of formula (III)

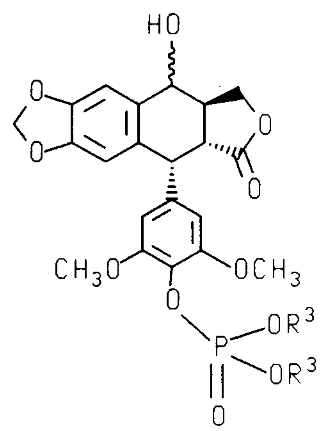

(III)

in the presence of a Lewis acid;

wherein

$R^1$ is a hydroxy protecting group, or the two $R^1$

groups together represent $C_{1-5}$alkylidene;

$R^2$ is a hydroxy protecting group; and

$R^3$ is a phosphate protecting group.

**12.** A process according to Claim 11 for preparing a compound of formula (I) wherein the two $R^1$ groups represent $C_{1-5}$alkylidene; $R^2$ is selected from the group consisting of benzyl, $C_{1-3}$alkanoyl, halogenated $C_{1-3}$alkanoyl, $C_{1-3}$alkoxycarbonyl and halogenated $C_{1-3}$alkoxycarbonyl; and $R^3$ is selected from the group consisting of phenyl, substituted phenyl, benzyl, substituted benzyl, and 2,2,2-trichloroethyl.

**13.** A process according to Claim 11 for preparing a compound of formula (I) wherein the two $R^1$ groups represent ethylidene, $R^2$ is 2,2,2-trichloroethoxycarbonyl, and $R^3$ is phenyl or benzyl.

**14.** A process according to Claim 11 wherein said Lewis acid is boron trifluoride etherate.

**15.** A process according to Claim 12 wherein said Lewis acid is boron trifluoride etherate.

**16.** A process according to Claim 13 wherein said Lewis acid is boron trifluoride etherate.

**17.** A compound having the formula

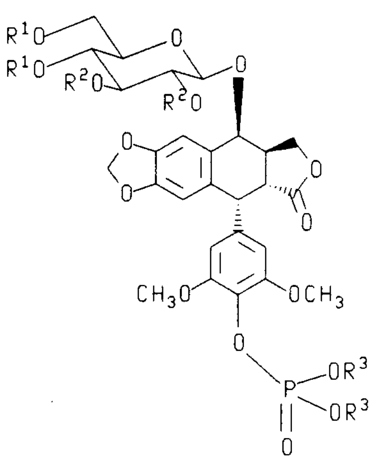

(I)

wherein

$R^1$ is a hydroxy protecting group, or the two $R^1$ groups together represent $C_{1-5}$alkylidene;

$R^2$ is a hydroxy protecting group; and

$R^3$ is a phosphate protecting group.

**18.** A compound according to Claim 17 wherein the two $R^1$ groups together represent $C_{1-5}$alkylidene, $R^2$ is selected from the group consisting of benzyl, $C_{1-3}$alkanoyl, halogenated $C_{1-3}$alkanoyl, $C_{1-3}$alkoxycarbonyl and halogenated $C_{1-3}$alkoxycarbonyl; and $R^3$ is selected from the group consisting of phenyl, substituted phenyl, benzyl, substituted benzyl, and 2,2,2-trichloroethyl.

**19.** A compound according to Claim 17 wherein the two $R^1$ groups together represent ethylidene, $R^2$ is 2,2,2-trichloroethyoxycarbonyl, and $R^3$ is phenyl or benzyl.

**20.** A compound having the formula

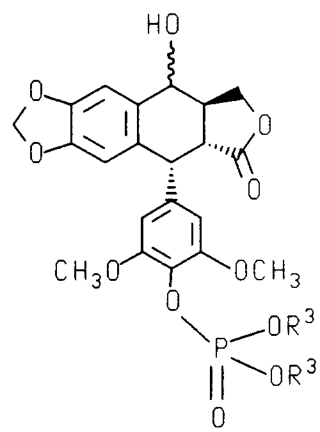

(III)

wherein $R^3$ is a phosphate protecting group.

**21.** A compound according to Claim 20 wherein $R^3$ is selected from the group consisting of phenyl, substituted phenyl, benzyl, substituted benzyl, and 2,2,2-trichloroethyl.

**22.** A compound according to claim 20 wherein $R^3$ is phenyl or benzyl.

European Patent Office

# EUROPEAN SEARCH REPORT

Application Number

EP 92106655.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US - A - 4 958 010 (J.F. KADOW et al.) * Examples 13-15; claims 1-25 * -- | 1-22 | C 07 H 15/20<br>C 07 H 17/04<br>C 07 F 9/12 |
| D,A | US - A - 4 904 768 (M.G. SAULNIER et al.) * Claims * -- | 1-19 | |
| A | US - A - 4 547 567 (H. UMEZAWA et al.) * Column 3, line 51 - column 4, line 43 * ---- | 20-22 | |
| | | | TECHNICAL FIELDS SEARCHED (Int. Cl.5) |
| | | | C 07 H 15/00<br>C 07 H 17/00<br>C 07 H 11/00<br>C 07 F 9/00 |

The present search report has been drawn up for all claims

| Place of search | Date of completion of the search | Examiner |
|---|---|---|
| VIENNA | 29-06-1992 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)